# EUROPEAN PATENT APPLICATION

(11) **EP 4 151 179 A1**
(43) Date of publication of application: **22.03.2023**
(21) Application number: 21912550.7
(22) Date of filing: 25.02.2021
(51) Int. Cl.: A61F 2/08, A61B 17/04, A61B 17/56

(54) **MEDICAL LOOP AND MANUFACTURING METHOD THEREFOR**

(30) Priority: 31.12.2020 CN 202011637928
(71) Applicant: Shanghai Orthopair Medical Co., Ltd., Shanghai 201201 (CN)
(72) Inventor: NIE, Jiali, Shanghai 201201 (CN); WANG, Yuanqiang, Shanghai 201201 (CN); YUAN, Zheng, Shanghai 201201 (CN)
(74) Representative: Bayramoglu et al.
(86) International application number: PCT/CN2021/077883
(87) International publication number: WO 2022/141780

(57) **Abstract**

Provided by the present invention is a medical loop, comprising a fixing plate and at least one suture, wherein the fixing plate is provided with at least two threading holes, and the first suture is wound to form a first coil wherein two free ends of the first coil are configured to pass through the threading holes, and the first coil is connected to the fixing plate to form a first fixing portion and a second fixing portion adapted to fix a graft, so that the first coil can automatically adjust the lengths of the first fixing portion and the second fixing portion under the action of the graft; wherein two free ends of the first coil are respectively defined as a first telescopic end and a first fixed end, the first telescopic end and the first fixed end are movably connected to form a first adjustable portion, so that the length of the first coil can be adjusted through the first adjustable portion under the action of artificial external force, and the first coil cannot be lengthened and retreated due to the pulling force of the graft when no artificial external force is applied. A manufacturing method of the medical loop is further provided by the present invention.

## Description

### FIELD OF TECHNOLOGY

The present invention relates to the field of medical devices, and more specifically, to a medical loop and a manufacturing method thereof.

### BACKGROUND

Loops are used for fixing ligaments or tendons and bones in orthopedic reconstruction, and are commonly used for anterior and posterior cruciate ligament reconstruction of the knee joints, acromioclavicular joint dislocation fixation and like. For example, in cruciate ligament surgery, bone tunnels need to be drilled in femur and tibia respectively, and a ligament graft without bone blocks at both ends is secured in the bone tunnels of the femur and tibia using a titanium plate with a loop and screws.

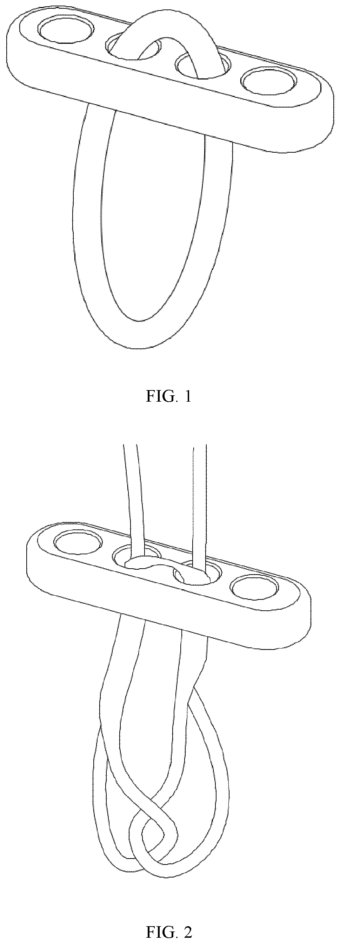

In the cruciate ligament reconstruction of the knee joints, the ligament graft should fill the femoral tunnel as completely as possible, and existing titanium plates with the loop are divided into a length-fixed titanium plate with a loop and an adjustable titanium plate with a loop. FIG. 1 is a structure diagram of a fixed-length titanium plate with a loop in the prior art. Referring to FIG. 1, a coil of the fixed-length titanium plate with the loop is usually of fixed specifications in different sizes, has no ability of adjusting the length of the coil, needs measurement and calculation before use, and is hard to completely fill the femoral tunnel, which may increase unnecessary surgery time and has surgical risks, and requires more space in the operating room for storage. FIG. 2 is a structure diagram of an adjustable titanium plate with a loop in the prior art. Referring to FIG. 2, the adjustable titanium plate with the loop can solve the problem of the above fixed-length titanium plate with the loop. However, the product currently available on the market is not conducive to surgery due to fact that the reaction force of tendon and other grafts makes the coil easily retreat; and the processing is complicated, and requires special equipment for winding and weaving to meet the same mechanical strength as the fixed-length titanium plate with the loop, resulting in high cost.

The patent application with publication number of CN108392235A discloses a loop used for a titanium plate with a loop, and in particular a loop for a titanium plate with the loop and a weaving process of the loop. The loop is woven to form a continuous ring by a cored woven rope, the cross section of the cored woven rope is oval, and a core yarn is arranged in the cored woven rope. The cored woven rope comprises a woven segment A, a woven segment B and a non-woven segment, wherein the woven segment A and the woven segment B are respectively defined as two free ends of the cored woven rope. One end of the non-woven segment is connected to the woven segment A, and the other end of the non-woven segment is connected to the woven segment B. An end A of the woven segment A threads in a hollow interior of the woven segment B to form a nesting portion A, and an end B of the woven segment B threads in a hollow interior of the woven segment A to form a nesting portion B. Compared with direct adhesive bonding or knotting fixation, the loop can overcome the defects that an existing medical suture loop is not firm in fixation and the like, and irritation to tissues is smaller. However, under the reaction force of a graft, an outer line sleeve of the cored woven rope is easy to deform, and a line core is relatively small in deformation, which is not conducive to the surgery; and the loop of the above invention belongs to fixed-length loops, which has no ability of adjusting the length of the coil and requires measurement and calculation before use; there is only one suture segment for fixing the graft, so that the material requirement of the suture is higher to meet the medical mechanical strength; and meanwhile, the cored woven rope is too thick to be convenient for clinical use.

Therefore, it is necessary to design a novel medical loop and a manufacturing method thereof to avoid the problems in the prior art.

### SUMMARY

An objective of the present invention is to provide a medical loop and a manufacturing method thereof to solve the problems that in the prior art the length of a coil cannot be adjusted and the coil is easy to be retreated under the reaction force of a graft, leading to problems to the operation in the surgery.

To achieve of the objective, a medical loop is provided, comprising a fixing plate and at least one suture, the fixing plate is provided with at least two threading holes, the suture is wound to form a first coil wherein two free ends of the first coil are configured to pass through the threading holes, and the first coil is connected to the fixing plate to form a first fixing portion and a second fixing portion adapted to fix a graft; two free ends of the first coil are respectively defined as a first telescopic end and a first fixed end, and the first telescopic end and the first fixed end configured to be movably connected therewith to form a first adjustable portion.

The medical loop of the present invention has the following beneficial effects: the two ends of the first coil are configured to pass through the threading holes, and the first coil is connected to the fixing plate to form the first fixing portion and the second fixing portion adapted to fix the graft, so that the first fixing portion and the second fixing portion can share pulling force of the graft, the overall stress strength of the medical loop is increased, the medical loop becomes firmer, and this can meet clinical requirements of structural mechanics and be suitable for the grafts with different pulling force. Moreover, the first coil can be automatically slidable to adjust the length of the first fixing portion and the length of the second fixing portion under the action of the graft, thus guaranteeing that the length of the first fixing portion can be the same as the length of the second fixing portion, no redundant calculation is required during surgery, the surgery time is shortened, and the surgical risk is reduced. The two ends of the first coil are respectively defined as the first telescopic end and the first fixed end, the first telescopic end and the first fixed end are fixedly connected to form the first adjustable portion, so that the length of the first coil can be adjusted through the first adjustable portion under the action of the artificial external force, i.e., the overall length of the first fixing portion and the second fixing portion, and meanwhile, the first adjustableportion forms locking force to the first telescopic and the first fixed end; moreover, as the first fixing portion and the second fixing portion are combined to fix the graft together, the length of the first coil cannot be changed due to the action of the pulling force of the graft, i.e., the first coil cannot be lengthened and retreated due to the action of the pulling force of the graft when no artificial external force is applied, the smooth surgery is ensured, and the surgical risk is reduced.

Preferably, the suture is further wound to form a second coil, two free ends of the second coil are respectively defined as a second telescopic end and a second fixed end, the second telescopic end and the second fixed end configured to pass through the threading holes, and the first coil forms a nested structure with the second coil to achieve the connection to the fixing plate. The beneficial effects include: the first coil, which mainly bears the acting force of the graft, is prevented from being directly connected to the fixing plate, and the damage to the first coil by the fixing plate is reduced.

Preferably, the at least one suture comprises one continuous suture, and the first coil and the second coil are woven by the continuous suture passing through the threading holes of the fixing plate. The beneficial effects include: a suture segment between the first coil and the second coil is continuous and becomes firmer and more durable, thus preventing this connection suture segment of the first coil and the second coil from being broken to affect the use thereof.

Preferably, the first coil and the second coil are respectively woven by two sutures of the at least one suture to pass through the threading holes of the fixing plate. The beneficial effects include: the weaving is simple and convenient.

Preferably, the first fixed end is fixedly connected to the second fixed end. The beneficial effects include: the stress strength of the first coil and the second coil is increased.

Preferably, the first telescopic end and the first fixed end are configured to pass through the common threading hole. The beneficial effects include: the friction force between the two ends of the first coil and the fixing plate is reduced, the number of the threading holes is reduced, and then the size of the fixing plate is reduced.

Preferably, the first telescopic end and the first fixed end are configured to respectively pass through the different threading holes, and the first telescopic end and the first fixed end are movably connected on the first side of the fixing plate to form the first adjustable portion. The beneficial effects include: the contact areas between the first telescopic end and the fixing plate and between the first fixed end and the fixing plate are increased, and the tension between the first telescopic end and the first fixed end is increased, thus increasing the friction force between the first telescopic end and the fixing plate and between the first fixed end and the fixing plate and making the anti-retreating effect better.

Preferably, the second telescopic end and the second fixed end are configured to pass through the common threading hole, and the second telescopic end and the second fixed end are movably connected to form a second adjustable portion. The beneficial effects include: the length of the second coil can be adjusted through the second adjustable portion, so that the length of the second coil can be adjusted and increased through the second adjustable portion under the action of artificial external force, and the first coil can be automatically sidable on the second coil under the action of the graft to adjust the length of the first fixing portion and the length of the second fixing portion, thus guaranteeing that the length of the first fixing portion is the same as the length of the second fixing portion.When the length of the first fixing portion and the length of the second fixing portion are adjusted to be the same, the length of the second coil can be adjusted and reduced through the second adjustable portion, so that the second coil is contracted to lock the first coil. Due to the locking force, the friction force between the first coil and the second coil is increased, then the first coil cannot be slidable and adjustable automatically, thus guaranteeing that the length of the first fixing portion and the length of the second fixing portion remain unchanged. Moreover, when the second coil is subjected to the action of artificial pulling force, and part of the nested structure formed by the first coil and the second coil is pulled into the threading hole, the nested structure is bound into the threading hole to increase the friction area between the first coil and the second coil, thus providing more friction force to further guarantee that the first coil cannot be slidable and adjustable automatically and to be better to guaranteeing that the length of the first fixing portion and the length of the second fixing portion can remain unchanged during use or during the day of clinical adjustment. And meanwhile, the second telescopic end and the second fixed end are configured to pass through the common threading hole, so that the friction force between the two ends of the second coil and the fixing plate is reduced, the number of the threading hole is reduced, and then the size of the fixing plate is reduced. Moreover, the first coil is locked onto the second coil and is not in contact with the fixing plate, the damage to the first coil by the fixing plate is reduced.

Preferably, the second telescopic end and the second fixed end are configured to pass through the common threading hole, and the second telescopic end and the second fixed end are fixedly connected. The beneficial effects include: the structure is simple and the weaving thereof is convenient.

Preferably, the second telescopic end and the second fixed end are configured to respectively pass through the different threading holes, and the second telescopic end and the second fixed end are movably connected on the first side of the fixing plate to form a second adjustable portion. The beneficial effects include: the length of the second coil can be adjusted through the second adjustable portion, so that the length of the second coil can be adjusted and increased through the second adjustable portion under the action of artificial external force, and the first coil can be automatically slidable on the second coil under the action of the graft to adjust the length of the first fixing portion and the length of the second fixing portion, thus guaranteeing that the length of the fixing portion can be the same as the length of the second fixing portion. When the length of the first portion and the length of the second fixing portion are adjusted to be the same, the length of the second coil can be adjusted and reduced through the second adjustable portion, so that the second coil contracts to lock the first coil between the second coil and the fixing plate, and the first coil cannot be automatically slidable to adjust, thus guaranteeing that the length of the first fixing portion and the length of the second fixing portion remain unchanged. And meanwhile, the second telescopic end and the second fixed end are configured to respectively pass through different threading holes, so that the contact area between the second telescopic end and the fixing plate and between the second fixed and the fixing plate is increased, the tension between the second telescopic end and the second fixed end is increased, and thus the friction force between the second telescopic end and the fixing plate and between the second fixed and the fixing plate is increased.

Preferably, the second telescopic end and the second fixed end are configured to respectively pass through the different threading holes, and the second telescopic end and the second fixed end are fixedly connected. The beneficial effects include: the structure is simple and the weaving thereof is convenient.

Preferably, the second telescopic end is movably connected to the second fixed end through any one of a threading process, a nesting method, a fastening method, a clamping method and a binding method to form a second adjustable portion. The beneficial effects include: on the one hand, the second elastic portion forms the locking force on the second telescopic end and the second fixed end, so that the length of the second coil cannot change automatically under the action of the graft; on the other hand, the second elastic portion cannot make the second telescopic end completely lose moving capacity with respect to the second fixed end, so that the length of the second coil can be movably adjusted through the second adjustable portion under the action of artificial external force.

Preferably, the first coil forms at least one fixing portion on the first side of the fixing plate by passing through the threading holes so as to achieve the connection to the fixing plate. The beneficial effects include: the first coil can be automatically slidable between the threading holes of the fixing plate under the action of the graft to adjust the length of the first fixing portion and the length of the second fixing portion, thus guaranteeing that the length of the fixing portion can be the same as the length of the second fixing portion. Therefore, the first fixing portion and the second fixing portion can share the pulling force of the graft, no redundant calculation is required during surgery, the surgery time is shortened, and the surgical risk is reduced, and moreover, the structure is simple, the design is ingenious, and the production cost input is low.

Preferably, the first telescopic end is movably connected to the first fixed end through any one of a threading process, a nesting method, a fastening method, a clamping method and a binding method to form the first adjustable portion. The beneficial effects include: on the other hand, the first adjustable portion forms the locking force on the first telescopic end and the first fixed end, so that the length of the first coil cannot change automatically under the action of the graft; on the other hand, the first adjustable portion cannot make the first telescopic end completely lose the moving ability with respect to the first fixed end, so that the length of the first coil can be movably adjusted through the first adjustable portion under the action of the artificial external force.

Preferably, a manufacturing method of a medical loop is further provided, comprising:
S0, providing a fixing plate and at least one suture, wherein the fixing plate is provided with at least two threading holes; and
S 1, winding the suture to form the first coil, enabling two free ends of the first coil to pass through the threading holes, and connecting the first coil to the fixing plate to form the first fixing portion and the second fixing portion adapted to fix the graft, and movably connecting the first telescopic end of the first coil to the first fixed end of the first coil to form the first adjustable portion.

The manufacturing method of the medical loop of the present invention has the following beneficial effects: enabling two free ends of the first coil to pass through the threading holes, and the first coil is connected to the fixing plate to form the first fixing portion and the second fixing portion adapted to fix the graft, so that the first fixing portion and the second fixing portion can share the pulling force of the graft, the overall stress strength of the medical loop is increased, the medical loop becomes firmer, and this can meet the clinical requirements to the structure mechanics and suitable for the grafts with different pulling force. Furthermore, the first coil can automatically slide under the action of the graft to adjust the length of the first fixing portion and the length of the second fixing portion to guarantee that the length of the first fixing portion can be the same as that of the second fixation and no redundant calculation is required during surgery, thus the surgery time is shortened and the surgical risk is reduced. By enabling the first telescopic end of the first coil and the first fixed end of the first coil to be movably connected therewith to form the first adjustable portion, the length of the first coil, i.e., the total length of the first fixing portion and the second fixing portion, can be adjusted under the action of artificial external force through the first adjustable portion. And due to the first fixing portion and the second fixing portion combined to jointly or commonly fix the graft, the first adjustable portion cannot be loosened by the pulling force of the graft to cause the length of the first coil to change. That is to say, the first coil cannot be lengthened and retreated by the action of the pulling force of the graft when no artificial external force is applied, the smooth surgery is guaranteed and the surgical risk is reduced.

Preferably, the first coil is configured to form at least one fixing portion on the first side of the fixing plate by passing through the threading holes so as to achieve the connection to the fixing plate, then the step S1 specifically comprises:
S101, enabling one end of the suture through the threading holes to form at least one fixing portion on the first side of the fixing plate so as to achieve the connection to the fixing plate, and forming the first coil on the second side of the fixing plate; and
S102, enabling the first telescopic end of the first coil and the first fixed end of the first coil to be movably connnected therewith to form the first adjustable portion, which is located on the first side of the fixing plate, thus making the first coil form the first fixing portion and the second fixing portion adapted to fix the graft on the second side of the fixing plate. The beneficial effects include: the first coil can be automatically slidable between the threading holes of the fixing plate through the fixing portion under the action of the graft to adjust the length of the first fixing portion and the length of the second fixing portion, the length of the first fixing portion is guaranteed to be the same as the length of the second fixing portion, so that no redundant calculation is required during surgery, the surgery time is shortened, and the surgical risk is reduced; and moreover, the structure is simple, the design is ingenious, and the product cost input is low.

Preferably, the suture is further wound to form a second coil, the first coil forms a nested structure with the second coil to achieve the connection to the fixing plate, and the first coil and the second coil are woven by one continuous suture passing through the threading holes of the fixing plate, then the step S1 specifically comprises:
S 111, enabling the continuous suture to pass through the threading holes and to form the first coil on the second side of the fixing plate, and movably connecting the first telescopic end of the first coil to the first fixed end of the first coil to form the first adjustable portion; and
S112, enabling the first fixed end to pass through the threading hole from the first side of the fixing plate and further to pass through the first coil to form the second coil, which is configured to forms the nested structure with the first coil; movably connecting the second telescopic end of the second coil to the second fixed end of the second coil to form the second adjustable portion or fixed connection, thus making the first coil form the first fixing portion and the second fixing portion adapted to fix the graft on the second side of the fixing plate. The beneficial effects include: the first coil for fixing the graft is prevented from being directly connected to the fixing plate, the damage to the first coil by the fixing plate is reduced, so that the suture between the first coil and the second coil is continuous, becomes firmer, and more durable, thus preventing a connection position of the first coil and the second coil from being broken to affect the use thereof.

Preferably, the two sutures of the at least one suture are respectively wound to form the first coil and the second coil, the first coil is woven by one suture according to the step S111, and when the first adjustable portion is formed, the following steps are executed sequentially:
S122, enabling one end of the other of the sutures to pass through the first coil in the step S111 to form the second coil, which is configured to form the nested structure with the first coil; and
S123, movably connecting the second telescopic end of the second coil to the second fixed end of the second coil to form a second adjustableportion or fixed connection, thus making the first coil form the first fixing portion and the second fixing portion adapted to fix the graft on the second side of the fixing plate. The beneficial effects include: the weaving thereof is simple and convenient.

### BRIEF DESCRIPTION OF THE DRAWINGS

FIG. 1 is a structure diagram of a fixed-length type titanium plate with a loop in the prior art;
FIG. 2 is a structure diagram of an adjustable type titanium plate with a loop in the prior art;
FIG. 3 is a structure diagram of a first medical loop of an embodiment of the present invention;
FIG. 4 is a structure diagram of a first adjustable portion in some embodiments of the present invention;
FIG. 5 is a structure diagram of a first adjustable portion in other embodiments of the present invention;
FIG. 6 is a structure diagram of a second medical loop of an embodiment of the present invention;
FIG. 7 is a structure diagram of a third medical loop of an embodiment of the present invention;
FIG. 8 is a structure diagram of a fourth medical loop of an embodiment of the present invention;
FIG. 9 is a structure diagram of a fifth medical loop of an embodiment of the present invention;
FIG. 10 is a diagram of a use state of a second medical loop of an embodiment of the present invention;
FIG. 11 is a sectional diagram of a partial structure of the second medical loop of an embodiment of the present invention;
FIG. 12 is a partial enlarged view of snapping a nested structure into a threading hole in FIG. 11;
FIG. 13 is a structure diagram of a fixing plate of an embodiment of the present invention;
FIG. 14 is a structure diagram formed in a manufacturing process of a medical loop of an embodiment of the present invention.

### DESCRIPTION OF THE EMBODIMENTS

In order to make objectives, technical solutions, and advantages of the present invention clearer, the technical solutions in the present invention are described clearly and completely in the following with reference to accompanying drawings in the embodiments of the present invention. Apparently, the described embodiments are only part rather than all of the embodiments of the present invention. Based on the embodiments of the present invention, all the other embodiments obtained by those of ordinary skill in the art without inventive effort are within the scope of the present invention. Unless otherwise mentioned, all technical and scientific terms used herein have the same meaning as commonly understood by one of ordinary skill in the art to which the invention belong. "Comprise", "include" and the like are intended to denote the element or object before the word is included in the listed element or object and thereof equivalents after the word, instead of excluding other elements or objects.

To solve the problems in the prior art, a medical loop is provided by an embodiment of the present invention, the medical loop comprising a fixing plate and at least one suture, wherein the fixing plate is provided with at least two threading holes, the suture is wound to form a first coil wherein two free ends of the first coil are configured to pass through the at least two threading holes, and the first coil is connected to the fixing plate to form a first fixing portion and a second fixing portion adapted to fix a graft, so that the first fixing portion and the second fixing portion can share the pulling force of the graft, the overall stress strength of the medical loop is increased, the medical loop becomes firmer, and this can meet the clinical requirements to structure mechanics and be suitable for grafts with different pulling force. Furthermore, the first coil can be automatically slidable under the action of the graft to adjust the length of the first fixing portion and the length of the second fixing portion to guarantee that the length of the first fixing portion can be the same as that of the second fixing portion and no redundant calculation is required during surgery, thus the surgery time is shortened and the surgical risk is reduced. Two free ends of the first coil are respectively defined as the first telescopic end and the first fixed end, the first telescopic end and the first fixed end are movably connected therewith to form the first adjustable portion, so that the length of the first coil , i.e., the overall length of the first fixing portion and the second fixing portion, can be adjusted under the action of the artificial external force through the first adjustable portion, and meanwhile, the first adjustable portion is also able to form a locking force to the first telescopic and the first fixed end. And due to the first fixing portion and the second fixing portion combined to fix the graft together, the length of the first coil cannot be changed due to the action of the pulling force of the graft. That is the first coil cannot be lengthened and retreated due to the action of the pulling force of the graft when no artificial external force is applied, thus the smooth surgery is ensured and the surgical risk is reduced.

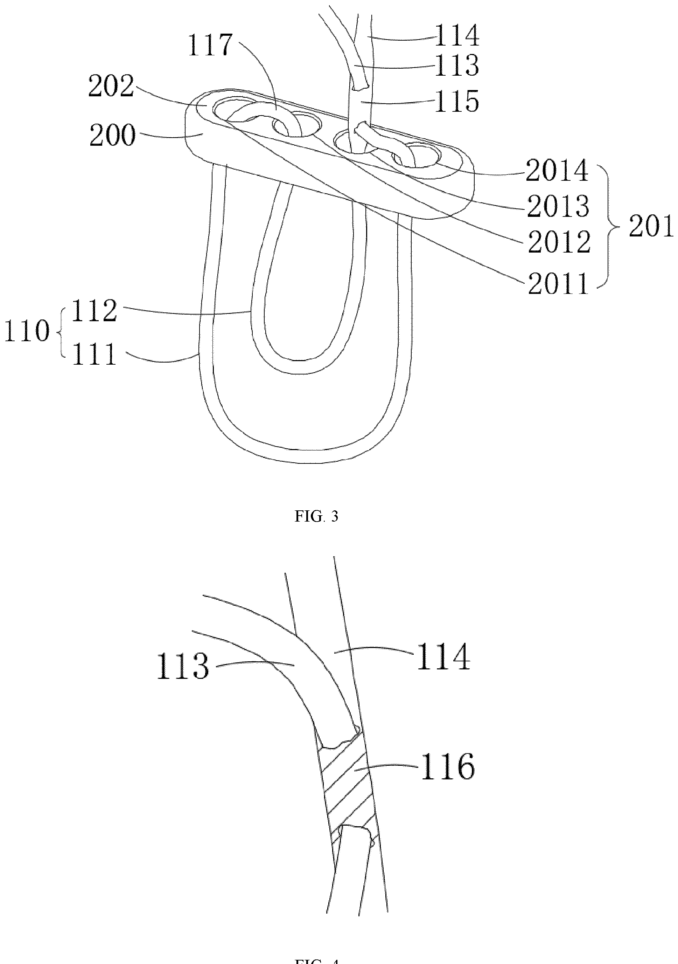

FIG. 3 is a structure diagram of a first medical loop of an embodiment of the present invention. In some embodiments of the present invention, referring to FIG. 3, the medical loop comprises a fixing plate 200 and a continuous suture (not labeled in figure), the fixing plate 200 is provided with at least two threading holes, the continuous suture (not labeled in figure) is wound to form a first coil 110 wherein two free ends of the first coil 110 are respectively defined as a first telescopic end 113 and a first fixed end 114, the first telescopic end 113 and the first fixed end 114 are configured to respectively pass through the different threading holes 201, and the first coil 110 forms at least one fixing portion 117 on a first side 202 of the fixing plate 200 by passing through the plurality of threading holes 201 so as to achieve the connection to the fixing plate 200, and forms a first fixing portion 111 and a second fixing portion 112 adapted to fix a graft; and the first telescopic end 113 and the first fixed end 114 are movably connected therewith on the first side 202 of the fixing plate 200 to form a first adjustable portion 115.

In some specific embodiments of the present invention, referring to FIG. 3, the first coil 110 forms at least one fixing portion 117 on the first side 202 of the fixing plate 200 by passing through the adjacent threading holes of the plurality of threading holes 201 so as to achieve the connection to the fixing plate 200. The threading holes 201 comprises a first threading hole 2011, a second threading hole 2012, a third threading hole 2013 and a fourth threading hole 2014 arranged in sequence. The first coil 110 forms one fixing portion 117 on the first side 202 of the fixing plate 200 by sequentially passing through the first threading hole 2011 and the second threading hole 2012 so as to achieve the connection to the fixing plate 200. And the first telescopic end 113 and the first fixed end 114 are configured to respectively pass through the fourth threading hole 2014 and the third threading hole 2013 and are movably connected therewith on the first side 202 of the fixing plate 200 to form the first adjustable portion 115, thus forming the first fixing portion 111 and the second fixing portion 112 adapted to fix the graft on the second side (not shown in figure) of the fixing plate 200. The first coil 110 can be automatically slidable under the action of the graft between the threading holes 201 of the fixing plate 200 through the fixing portion 117 so as to adjust the length of the first fixing portion 111 and the length of the second fixing portion 112, thus guaranteeing that the length of the first fixing portion 111 can be the same as that of the second fixing portion 112. Therefore, the first fixing portion 111 and the second fixing portion 112 can share the pulling force of the graft, no redundant calculation is required during surgery and the surgery time is shortened, thus the surgical risk is reduced. Further, the structure is simple, the design is ingenious, and the production cost input is low.

In some other embodiments of the present invention, the first coil forms at least one fixing portion on the first side of the fixing plate by passing through the plurality of threading holes so as to achieve the connection to the fixing plate, and the fixing portion is provided across at least one of the threading holes.

In some embodiments of the present invention, the first telescopic end is movably connected to the first fixed end by way of any one of a threading process, a nesting method, a fastening method, a clamping method, and a binding method to form the first adjustable portion. The first adjustable portion also forms the locking force on the first telescopic and the first fixed end, so that the length of the first coil cannot automatically change under the action of the graft. On the other hand, the first adjustable portion cannot make the first telescopic end completely lose moving capacity with respect to the first fixed end, so that the length of the first coil can be movably adjusted under the action of artificial external force through the first adjustable portion .

FIG. 4 is a structure diagram of a first elastic portion in some embodiments of the present invention. In some specific embodiments of the present invention, referring to FIG. 4, the first fixed end 114 is provided with a first nesting portion 116, the first telescopic end 113 is configured to pass through the first nesting portion 116 to be movably connected to the first fixed end 114 so as to form the first adjustable portion (not labeled in figure), the first telescopic end 113 can pass through the first nesting portion 116 simply and easily, and the operation is simple and convenient, thus facilitating the manufacturing of the medical loop.

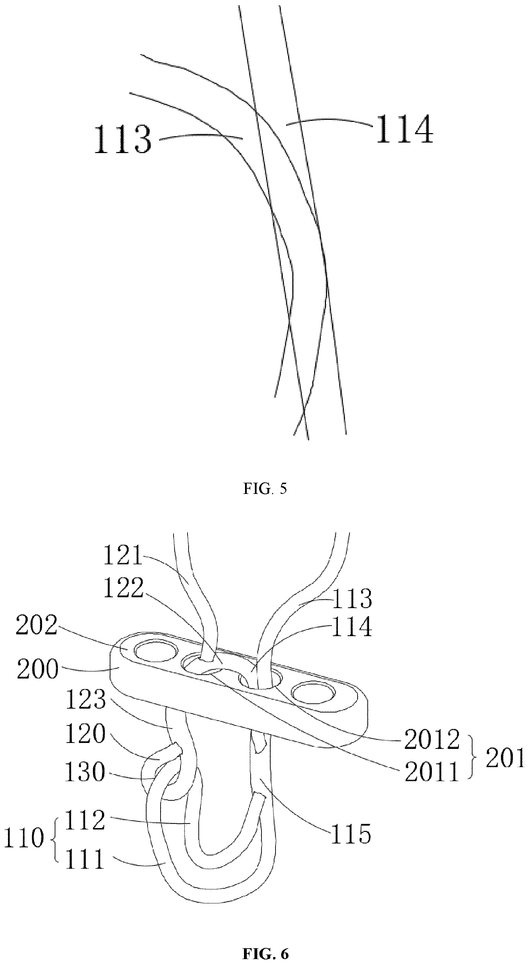

FIG. 5 is a structure diagram of the first elastic portion in some other embodiments of the present invention. In some other specific embodiments of the present invention, referring to FIG. 5, the first telescopic end 113 is movably connected to the first fixed end 114 through a threading process to form the first adjustable portion (not labeled in figure). Due to the own weaving process of the suture, the outer surface roughness of the suture is high, and the line hole at the interior of the suture has a rough inner surface; a suture segment of the first telescopic end 113 penetrates into part of a suture segment of the first fixed end 114 by means of the threading process, so that the outer surface of the suture of the first telescopic end 113 is in interference fit with the line hole of the suture at the first fixed end 114, thus providing an anti-retreating effect through the friction force therebetween.

In some further specific embodiments of the present invention, the first telescopic end is movably connected to the first fixed end through a binding method to form the first adjustable portion; the first telescopic end is movably connected to the first fixed end through the binding method, so that the first adjustable portion at the joint of the first telescopic end and the first fixed end has a certain friction force thereof with no lose of the moving capacity, and during surgery, the length of the first coil can be adjusted through the first adjustable portion under the artificial external force. The specific binding method is the conventional means of this field, unnecessary details are not given herein.

In still some other specific embodiments of the present invention, the first telescopic end and the first fixed end are both provided with fasteners, the first telescopic end is movably connected to the first fixed end through a fastening method to form the first adjustable portion, the specific structure of the fastener is the conventional means of this field, and unnecessary details are not given herein. The fastener forms the locking force on the first telescopic end and the first fixed end and cannot make the first telescopic end completely lose the moving capacity with respect to the first fixed end, so that the length of the first coil can be adjusted through the first adjustable portion under the artificial external force during surgery.

In yet some other specific embodiments of the present invention, the first telescopic end and the first fixed end are both provided with clamps, the first telescopic end is movably connected to the first fixed end through a clamping method to form the first adjustable portion, the specific structure of the clamp is the conventional means of this field, and unnecessary details are not given herein. The clamp cannot make the first telescopic end completely lose the moving capacity with respect to the first fixed end and forms the locking force on the first telescopic end and the first fixed end, so that the length of the first coil can be adjusted through the first elastic portion under the artificial external force during surgery.

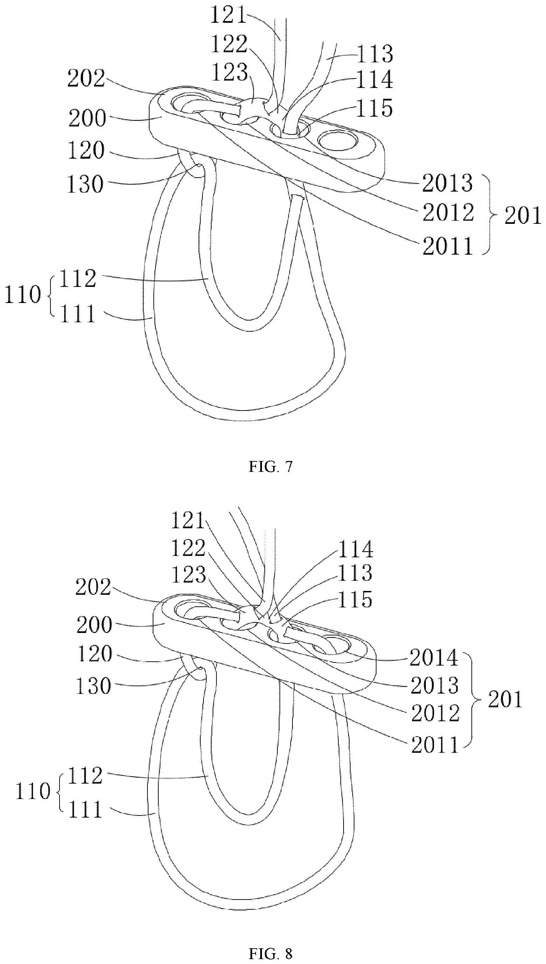

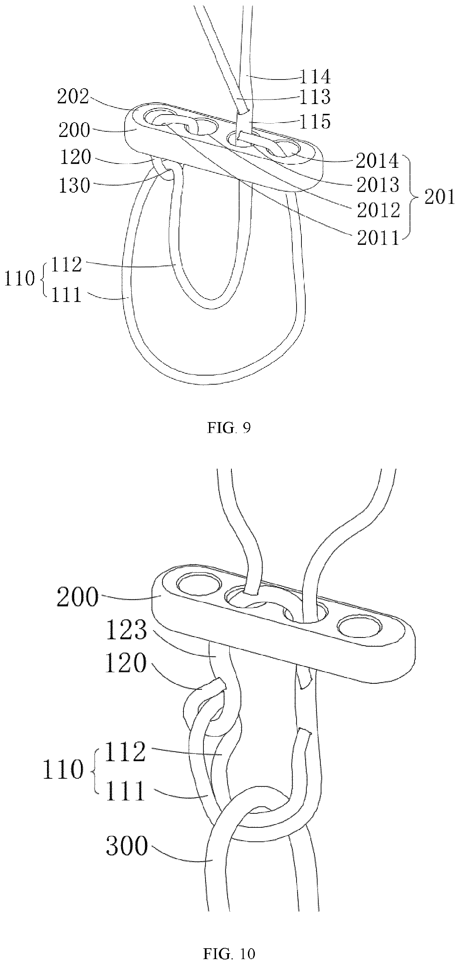

FIG. 6 is a structure diagram of a second medical loop of an embodiment of the present invention. FIG. 7 is a structure diagram of a third medical loop of an embodiment of the present invention. FIG. 8 is a structure diagram of a fourth medical loop of an embodiment of the present invention. FIG. 9 is a structure diagram of a fifth medical loop of an embodiment of the present invention.

In some embodiments of the present invention, referring to FIG. 6, FIG. 7, FIG. 8 and FIG. 9, the suture is further wound to form a second coil 120. Two free ends of the second coil 120 are respectively defined as a second telescopic end 121 and a second fixed end 122. The second telescopic end 121 and the second fixed end 122 are configured to respectively pass through the two threading holes, and the first coil 110 forms a nested structure 130 with the second coil 120 so as to achieve the connection to the fixing plate 200. The first coil 110 is connected to the fixing plate 200 through the second coil 120 so as to form the first fixing portion 111 and the second fixing portion 112 for fixing the graft. This can be prevented from enabling the first coil 110, which mainly bears the acting force of the graft, to be directly connected to the fixing plate 200, and the damage to the first coil 110 by the fixing plate 200 is reduced.

In the embodiments of the present invention, the first telescopic end 113 and the first fixed end 114 are respectively two free ends of the first coil 110, the first telescopic end 113 comprises a suture segment of one free end of the first coil 110 configured to penetrate through the threading hole, a suture segment which forms the first adjustable portion 115, and a suture segment which is located at the first side 202 of the fixing plate 200. The first fixed end 114 comprises a suture segment of the other free end of the first coil 110 configured to penetrate through the threading hole 201, a suture segment forming the first adjustable portion 115, and a suture segment located at the first side 202 of the fixing plate 200. The first fixing portion 111 and the second fixing portion 112 are respectively formed by two suture segments of the first coil 110 divided by the fixing plate or the second coil, wherein the first fixing portion 111 is one suture segment of the first coil 110 located at the second side (not labeled in figure) of the fixing plate 200, and the second fixing portion 112 is another suture segment of the first coil 110 located at the second side (not labeled in figure) of the fixing plate 200. The second telescopic end 121 and the second fixed end 122 are respectively two free ends of the second coil 120, the second telescopic end 121 comprises a suture segment of one free end of the second coil 120 configured to penetrate through the threading hole, a suture segment which forms the second adjustable portion 123, and a suture segment which is located at the first side 202 of the fixing plate 200. The second fixed end 122 comprises a suture segment of the other free end of the second coil 120 configured to penetrate through the threading hole 201, a suture segment which forms the second elastic portion 123, and a suture segment which is located at the first side 202 of the fixing plate 200.

In some embodiments of the present invention, referring to FIG. 6, FIG. 7 and FIG. 8, the at least one suture (not labeled in figure) comprises one continuous suture (not shown in figure), the first coil 110 and the second coil 120 are woven by a common continuous suture configured to pass through the threading holes 201 of the fixing plate 200, the suture segment between the first coil 110 and the second coil 120 is of a continuous suture, which becomes firmer and more durable, thus preventing this connection suture segment of the first coil 110 and the second coil 120 from being broken to affect the use thereof.

In some other embodiments of the present invention, referring to FIG. 9, the first coil 110 and the second coil 120 are respectively woven by two sutures (not labeled in figure) of the at least one suture configured to pass through the threading holes 201 of the fixing plate 200, and the weaving of the first coil 110 and the second coil 120 is simple and convenient.

In some further embodiments of the present invention, the first coil and the second coil are respectively woven by two sutures of the at least one suture configured to pass through the threading holes of the fixing plate, and the first fixed end is fixedly connected to the second fixed end, thus increasing the stress strength of the first coil and the second coil.

In some embodiments of the present invention, the first fixed end and the second fixed end have a fixed connection, which is achieved through a bonding method or a binding method.

In some other embodiments of the present invention, the first fixed end and the second fixed end are arranged to be of a hard knot structure so as to achieve fixed connection.

In some further embodiments of the present invention, the first fixed end is woven into the suture nested in the second fixed end to achieve fixed connection, or the second fixed end is woven into the suture nested in the first fixed end to achieve fixed connection.

In some embodiments of the present invention, referring to FIG. 6 and FIG. 7, the at least one suture comprises a continuous suture (not labeled in figure), the first coil 110 and the second coil 120 are woven by the continuous suture (not labeled in figure) configured to pass through the threading holes 201 of the fixing plate 200, the first telescopic end 113 and the first fixed end 114 is configured to pass through the same threading hole 201, so that the friction force between the two ends of the first coil 110 and the fixing plate 200 is reduced, the number of the threading holes 201 is reduced, and then the size of the fixing plate 200 is reduced.

In some other embodiments of the present invention, the first coil and the second coil are respectively woven by two sutures configured to pass through the threading holes of the fixing plate, the first fixed end is fixedly connected to the second fixed end, and the first telescopic end and the first fixed end are configured to pass through the same or common threading hole.

In some embodiments of the present invention, referring to FIG. 8 and FIG. 9, the first telescopic end 113 and the first fixed end 114 are configured to respectively pass through different threading holes 201, and the first telescopic end 113 and the first fixed end 114 are movably connected therewith on the first side 202 of the fixing plate 200 to form the first adjustable portion 115, so that the contact areas between the first telescopic end 113 and the fixing plate 200 and between the first fixed end 114 and the fixing plate 200 are increased, the tension between the first telescopic end 113 and the first fixed end 114 is increased, the friction force between the first telescopic end 113 and the fixing plate 200 and between the first fixed end 114 and the fixing plate 200 are increased, and thus the anti-retreating effect is better.

In some embodiments of the present invention, referring to FIG. 6, the at least one suture comprises a continuous suture, the first coil 110 and the second coil 120 are woven by the continuous suture configued to pass through the threading holes 201 of the fixing plate 200, the second telescopic end 121 and the second fixed end 122 are configured to pass through the same threading hole 201, and the second telescopic end 121 and the second fixed end 122 are movably connected therewith to form a second adjustable portion 123.

In some other embodiments of the present invention, the first coil and the second coil are respectively woven by two sutures configured to pass through the threading holes of the fixing plate, the first fixed end is fixedly connected to the second fixed end, the second telescopic end and the second fixed end are configured to pass through the same or common threading hole, and the second telescopic end and the second fixed end are movably connected therewith to form the second adjustable portion.

In some embodiments of the present invention, the at least one suture comprises one continuous suture, the first coil and the second coil are woven by the continuous suture passing through the threading holes of the fixing plate, the second telescopic end and the second fixed end are configured to pass through the same or common threading hole, and the second telescopic end is fixedly connected to the second fixed end.

In some other embodiments of the present invention, the first coil and the second coil are respectively woven by two sutures passing through the threading holes of the fixing plate, the first fixed end is fixedly connected to the second fixed end, the second telescopic end and the second fixed end are configured to pass through the same or common threading hole, and the second telescopic end is fixedly connected to the second fixed end.

In some embodiments of the present invention, referring to FIG. 7 and FIG. 8, the second telescopic end 121 and the second fixed end 122 are configured to respectively pass through different threading holes 201, and the second telescopic end 121 and the second fixed end 122 are movably connected therewith on the first side 202 of the fixing plate 200 to form the second adjustable portion 123. The second telescopic end 121 and the second fixed end 122 are configured to respectively pass through different threading holes 201, so that the contact areas between the second telescopic end 121 and the fixing plate 200 and between the second fixed end 122 and the fixing plate 200 are increased, the tension between the second telescopic end 121 and the second fixed end 122 is increased, and the friction force between the second telescopic end 121 and the fixing plate 200 and between the second fixed end 122 and the fixing plate 200 is increased. Due to the second telescopic end 121 and the second fixed end 122 are movably connected on the first side 202 of the fixing plate 200 to form the second adjustable portion 123, the length of the second coil 120 can be adjusted through the second adjustable portion 123, i.e., the length of the second coil 123 can be adjusted and increased through the second adjustable portion 123 under the action of the artificial external force, and the first coil 110 under the action of the graft can be automatically slidable along the second coil 120 so as to adjust the length of the first fixing portion 111 and the length of the second fixing portion 112, thus guaranteeing that the length of the first fixing portion 111 can be the same as that of the second fixing portion 112. When the length of the first fixing portion 111 and the length of the second fixing portion 112 are adjusted to be the same, the length of the second coil 123 can be adjusted and reduced through the second adjustable portion 123, so that the second coil 123 can be contracted in order to enable the first coil 110 to be held by the second coil 120 and the fixing plate 200, and thus the first coil 110 cannot be automatically slidable to adjust, thus guaranteeing that the length of the first fixing portion 111 and the length of the second fixing portion 112 remain unchanged.

In some other embodiments of the present invention, referring to FIG. 9, the second telescopic end and the second fixed end are configure to respectively pass through different threading holes, and the second telescopic end is fixedly connected to the second fixed end, so that the structure is simple and the weaving is convenient.

In some embodiments of the present invention, the second telescopic end is fixedly connected to the second fixed end through a bonding method or a binding method.

In some other embodiments of the present invention, the second telescopic end and the second fixed end are arranged to be of a hard knot structure so as to achieve a fixed connection.

In some further embodiments of the present invention, the second telescopic end is woven into the suture of the second fixed end to achieve fixed connection, or the second fixed end is woven into the suture of in the second telescopic end to achieve fixed connection.

In some embodiments of the present invention, the second telescopic end is movably connected to the second fixed end through any one of a threading process, a nesting method, a fastening method, a clamping method or a binding method to form a second adjustable portion. On the one hand, the second adjustable portion forms the locking force on the second telescopic end and the second fixed end, so that the length of the second coil cannot change automatically under the action of the graft. On the other hand, the second adjustable portion cannot make the second telescopic end completely lose the moving capacity with respect to the second fixed end, so that the length of the second coil can be movably adjusted through the second adjustable portion under the action of artificial external force.

In some specific embodiments of the present invention, the second fixed end is provided with a second nesting portion, the second telescopic end is configured to pass through the second nesting portion to be movably connected to the second fixed end so as to form the second adjustable portion, and the second telescopic end can pass through the second nesting portion simply and easily, the operation is simple and convenient, thus facilitating the manufacturing of the medical loop.

In some other specific embodiments of the present invention, the second telescopic end is movably connected to the second fixed end through a threading process to form the second adjustable portion. Due to the own weaving process of the suture, the outer surface roughness of the suture is high, and the line hole at the interior of the suture is of a rough inner surface. Part of a suture segment of the second telescopic end penetrates into part of a suture segment of the second fixed end by means of the threading process, so that the outer surface of the suture of the second telescopic end is in interference fit with the line hole at the interior of the suture at the second fixed end, thus providing an anti-retreating effect through the friction force therebetween.

In some further specific embodiments of the present invention, the second telescopic end is movably connected to the second fixed end through a binding method to form the second adjustable portion; the second telescopic end is movably connected to the second fixed end through a binding method, so that the second adjustable portion at the joint of the second telescopic end and the second fixed end can have a certain friction force thereof with no lose of the moving capacity, and the length of the second coil can be adjusted through the second adjustable portion under the artificial external force during surgery. Due to the specific binding method is the conventional means of this field, no necessary details need to be given herein.

In still some specific embodiments of the present invention, the second telescopic end and the second fixed end are both provided with fasteners, the second telescopic end is movably connected to the second fixed end through a fastening method to form the second adjustable portion. Due to the specific structure of the fastener is the conventional means of this field, no necessary details need to be given herein. The fastener cannot make the second telescopic end completely lose the moving capacity with respect to the second fixed end and forms the locking force on the second telescopic end and the second fixed end, so that the length of the second coil can be adjusted under the artificial external force through the second adjustable portion during surgery.

In yet some other specific embodiments of the present invention, the second telescopic end and the second fixed end are both provided with clamps, the second telescopic end is movably connected to the second fixed end through a clamping method to form the second adjustable portion. Due to the specific structure of the clamp is the conventional means of this field, no necessary details need to be given herein. The clamp cannot make the second telescopic end completely lose the moving capacity with respect to the second fixed end and forms the locking force on the second telescopic end and the second fixed end, so that the length of the second coil can be adjusted under the artificial external force through the second adjustable portion during surgery.

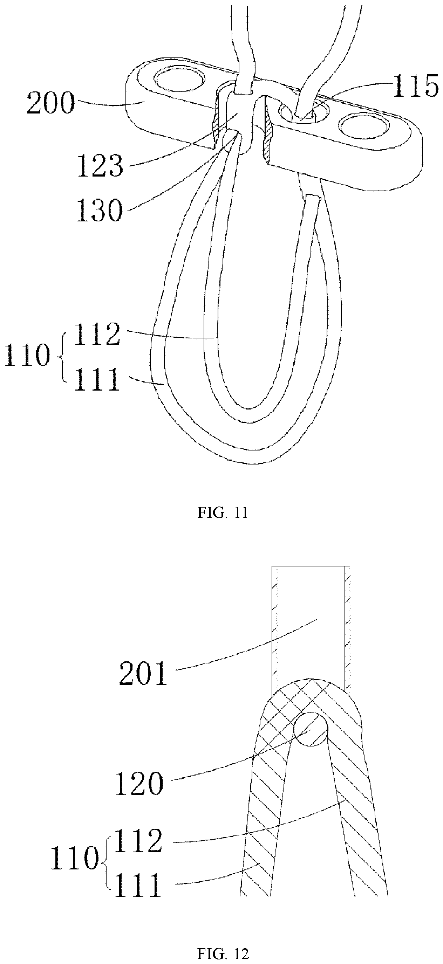

FIG. 10 is a diagram of a use state of a second medical loop of an embodiment of the present invention, FIG. 11 is a sectional diagram of a part of structure of the second medical loop of an embodiment of the present invention, and FIG. 12 is a partial enlarged view of snapping a nested structure into a threading hole in FIG. 11.

In some specific embodiments of the present invention, referring to FIG. 6 and FIG. 10, the medical loop comprises a fixing plate 200 and a continuous suture (not labeled in figure), the fixing plate 200 is provided with two threading holes 201, respectively defined as a first threading hole 2011 and a second threading hole 2012. The continuous suture (not labeled in figure) is wound to form a first coil 110 and a second coil 120 by passing through the threading holes of the fixing plate 200, wherein two free ends of the first coil 110 are configured to pass through the same or common threading hole 201, and two free ends of the second coil 120 are configured to pass through the same or common threading hole, and the threading hole 201 through which the two free ends of the second coil 120 are configured to pass is different from the threading hole 201 through which the two free ends of the first coil 110 are configured to pass. The two ends of the first coil 110 are respectively defined as a first telescopic end 113 and a first fixed end 114, and the first telescopic end 113 and the first fixed end 114 are movably connected to form a first adjustable portion 115 and to pass through the second threading hole 2012. The two free ends of the second coil 120 are respectively defined as a second telescopic end 121 and a second fixed end 122, and the second telescopic end 121 and the second fixed end 122 are movably connected to form a second adjustable portion 123 and to pass through the first threading hole 2011. The first coil 110 forms a nested structure 130 with the second coil 120 so as to achieve the connection to the fixing plate 200, and the first coil 110 is connected to the fixing plate 200 through the second coil 120 so as to form a first fixing portion 111 and a second fixing portion 112 adapted to fix a graft 300. the first fixed end 114 and the second fixed end 122 are on the continuous suture, and the first fixed end 114 and the second fixed end 122 are configured to pass through the different threading holes 201, thus enabling the first coil 110 and the second coil 120 to be sleeved on the fixing plate.

Specifically, referring to FIG. 10, the length of the second coil 120 can be adjusted through the second adjustable portion 123, so that the length of the second coil 120 can be adjusted and increased through the second adjustable portion 123 under the action of artificial external force, the first coil 110 can be automatically slidable on the second coil 120 under the action of the graft 300 so as to adjust the length of the first fixing portion 111 and the length of the second fixing portion 112, thus guaranteeing that the length of the first fixing portion 111 can be the same as the length of the second fixing portion 112. When the length of the first fixing portion 111 is adjusted to be the same as the length of the second fixing portion 112, the length of the second coil 120 can be adjusted and reduced through the second adjustable portion 123, so that the second coil 120 can be contracted to lock the first coil 110, the friction force between the first coil 110 and the second coil 120 is increased due to the locking force, the first coil 110 cannot be automatically slidable to adjust, thus guaranteeing that the length of the first fixing portion 111 and the length of the second fixing portion 112 remain unchanged. When the second coil 120 is subjected to the action of artificial pulling force, and the nested structure 130 formed by the first coil 110 and the second coil 120 can be pulled into the threading hole 201, referring to FIG. 11 and FIG. 12, the nested structure 130 is bound into the threading hole 201 to increase the friction area between the first coil 110 and the second coil 120, thus providing more friction force to further guarantee that the first coil 110 cannot be slidable and adjustable automatically and to be better to guaranteeing that the length of the first fixing portion 111 and the length of the second fixing portion 112 can remain unchanged during the day of clinical adjustment or during use.

In some other specific embodiments of the present invention, referring to FIG. 7, the medical loop comprises a fixing plate 200 and a continuous suture (not labeled in figure); the fixing plate 200 is provided with three threading holes 201, respectively defined as a first threading hole 2011, a second threading hole 2012, and a third threading hole 2013. The continuous suture (not labeled in figure) is wound to form a first coil 110 and a second coil 120 by passing through the threading holes 201 of the fixing plate 200, two free ends of the first coil 110 are configured to pass through the same or common threading hole 201, and the two free ends of the first coil 110 are respectively defined as a first telescopic end 113 and a first fixed end 114, the first telescopic end 113 and the first fixed end 114 are movably connected to form a first adjustable portion 115 and configured to pass through the third threading hole 2013. Two free ends of the second coil 120 are respectively defined as a second telescopic end 121 and a second fixed end 122, the second telescopic end 121 and the second fixed end 122 are configured to respectively pass through the first threading hole 2011 and the second threading hole 2012 and are movably connected to form a second adjustable portion 123. The first coil 110 forms a nested structure 130 with the second coil 120 so as to achieve the connection to the fixing plate 200, and the first coil 110 is connected to the fixing plate 200 through the second coil 120 so as to form a first fixing portion 111 and a second fixing portion 112 for fixing a graft, and the first fixed end 114 and the second fixed end 122 are of the continuous suture.

In some further specific embodiments of the present invention, referring to FIG. 8, the medical loop comprises a fixing plate 200 and a continuous suture (not labeled in figure); the fixing plate 200 is provided with four threading holes 201, respectively defined as a first threading hole 2011, a second threading hole 2012, a third threading hole 2013, and a fourth threading hole 2014. The continuous suture (not labeled in figure) is wound to form a first coil 110 and a second coil 120 by passing through the threading holes 201 of the fixing plate 200. Two free ends of the first coil 110 are respectively defined as a first telescopic end 113 and a first fixed end 114, the first telescopic end 113 and the first fixed end 114 are configured to respectively pass through the fourth threading hole 2014 and the third threading hole 2013 and are movably connected to form a first adjustable portion 115. Two free ends of the second coil 120 are respectively defined as a second telescopic end 121 and a second fixed end 122, the second telescopic end 121 and the second fixed end 122 are configured to respectively pass through the first threading hole 2011 and the second threading hole 2012 and are movably connected to form a second adjustable portion 123; the first coil 110 forms a nested structure 130 with the second coil 120 so as to achieve the connection to the fixing plate 200, and the first coil 110 is connected to the fixing plate 200 through the second coil 120 so as to form a first fixing portion 111 and a second fixing portion 112 for fixing a graft; and the first fixed end 114 and the second fixed end 122 are on the continuous suture.

In still some specific embodiments of the present invention, referring to FIG. 9, the medical loop comprises a fixing plate 200 and two sutures (not labeled in figure), the fixing plate 200 is provided with four threading holes 201, respectively defined as a first threading hole 2011, a second threading hole 2012, a third threading hole 2013 and a fourth threading hole 2014. One suture (not labeled in figure) in the two continuous sutures (not labeled in figure) is wound to form a first coil 110, two free ends of the first coil 110 are respectively defined as a first telescopic end 113 and a first fixed end 114, and the first telescopic end 113 and the first fixed end 114 are configured to respectively pass through the fourth threading hole 2014 and the third threading hole 2013 and are movably connected to form a first adjustable portion 115, while another suture (not labeled in figure) of the two continuous sutures (not labeled in figure) is wound to form a second coil 120, two free ends of the second coil 120 are respectively defined as a second telescopic end (not labeled in figure) and a second fixed end (not labeled in figure), the second telescopic end (not labeled in figure) and the second fixed end (not labeled in figure) are configured to respectively pass through the first threading hole 2011 and the second threading hole 2012 and are fixedly connected therewith, the first coil 110 forms a nested structure 130 with the second coil 120 so as to achieve the connection to the fixing plate 200, and the first coil 110 is connected to the fixing plate 200 through the second coil 120 so as to form a first fixing portion 111 and a second fixing portion 112 for fixing a graft.

In some embodiments of the present invention, the suture is any one of a silk suture, a catgut suture, a chemical synthesis suture, and a pure natural collagen suture, and the suture conforming to the stress strength is selected according to the clinic need for stress.

In some embodiments of the present invention, the suture is any one of a polymer polyethylene surgical suture, a nylon suture, a polyester suture, a polyamide suture, and a polypropylene suture.

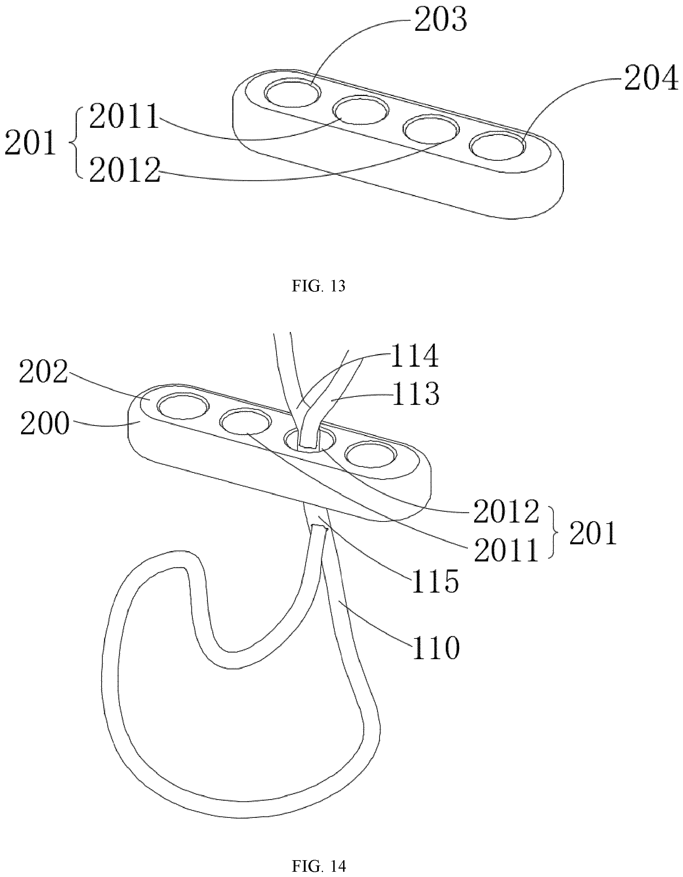

FIG. 13 is a structure diagram of a fixing plate of an embodiment of the present invention. In some better embodiments of the present invention, referring to FIG. 13, the fixing plate (not shown in figure) is further provided with at least one traction hole 203, the traction hole 203 is provided to facilitate the providing of a traction rope in clinic to pull the loop to an accurate position. In some preferred embodiments of the present invention, the threading hole can be temporarily taken as a traction hole for use.

In some better embodiments of the present invention, referring to FIG. 13, the fixing plate (not shown in figure) is further provided at least one loop overturning hole 204, and the loop overturning hole 204 is provided to facilitate the providing of a loop overturning line to overturn and adjust the fixing plate to an accurate angle and position. In some preferred embodiments of the present invention, the threading hole can be temporarily taken as a loop overturning line for use.

In some embodiments of the present invention, the threading holes of the fixing plate have the same aperture.

In some embodiments of the present invention, part or all of the threading holes of the fixing plate have apertures with diffrent diameters, thus facilitating a plurality of suture segments to pass through the same or common threading hole with a large aperture.

In some embodiments of the present invention, the fixing plate is any one of a titanium plate and a PEEK, the PEEK is poly-ether-ether-ketone, i.e., the PEEK is made of poly-ether-ether-ketone.

In some embodiments of the present invention, a manufacturing method of a medical loop is provided, comprising:
S0, providing a fixing plate and the at least one suture, wherein the fixing plate is provided with at least two threading holes; and
S1, winding the suture to form the first coil, enabling two free ends of the first coil to pass through the threading holes, and connecting the first coil to the fixing plate to form the first fixing portion and the second fixing portion adapted to fix the graft, and enabling the first telescopic end of the first coil and the first fixed end of the first coil to be movably connected therewith to form the first adjustable portion.

In some embodiments of the present invention, the first coil is configured to form at least one fixing portion on a first side of the fixing plate by passing through the plurality of threading holes so as to achieve the connection to the fixing plate, then the step S1 specifically comprises:
S101, enabling one free end of the suture to pass through the plurality of threading holes to form at least one fixing portion on the first side of the fixing plate so as to achieve the connection to the fixing plate, and forming the first coil on the second side of the fixing plate; and
S102, enabling the first telescopic end of the first coil and the first fixed end of the first coil to be movably connected to form the first adjustable portion, which is located on the first side of the fixing plate, thus making the first coil form the first fixing portion and the second fixing portion adapted to fix the graft on the second side of the fixing plate.

In some specific embodiments of the present invention, referring to FIG. 3, a manufacturing method of a medical loop is provided, comprising:
S10100, providing a fixing plate 200 and a continuous suture (not labeled in figure), wherein the fixing plate 200 is provided with four threading holes 201, and the threading holes 201 are respectively defined as a first threading hole 2011, a second threading hole 2012, a third threading hole 2013 and a fourth threading hole 2014 arranged in sequence;
S 10101, sequentially enabling one free end of the suture (not labeled in figure) to pass through the first threading hole 2011 and the second threading hole 2012 from a second side (not labeled in figure) of the fixing plate 200 to form a fixing portion 117 on a first side 202 of the fixing plate 200 so as to achieve the connection to the fixing plate 200, and forming the first coil 110 on the second side (not labeled in figure) of the fixing plate 200; and
S10202, after enabling a first telescopic end 113 of the first coil 110 and a first fixed end 114 of the first coil 110 to respectively pass through the fourth threading hole 2014 and the third threading hole 2013 from the second side (not labeled in figure) of the fixing plate 200, movably connecting the first telescopic end 113 to the first fixed end 114 on the first side 202 of the fixing plate to form a first adjustable portion 115, thus making the first coil 110 form a first fixing portion 111 and a second fixing portion 112 adapted to fix a graft on the second side (not labeled in figure) of the fixing plate 200.

In some other specific embodiments of the present invention, referring to FIG. 3, a manufacturing method of a medical loop is provided, comprising:
S10110, providing a fixing plate 200 and a continuous suture (not labeled in figure), wherein the fixing plate 200 is provided with four threading holes 201, and the threading holes 201 are respectively defined as a first threading hole 2011, a second threading hole 2012, a third threading hole 2013 and a fourth threading hole 2014 arranged in sequence;
S10111, sequentially enabling one free end of the suture (not labeled in figure) to pass through the third threading hole 2013, the second threading hole 2012 and the first threading hole 2011 from a first side 202 of the fixing plate 200 to form a fixing portion 117 on a first side 202 of the fixing plate 200 so as to achieve the connection to the fixing plate 200, and forming the first coil 110 on the second side (not shown in figure) of the fixing plate 200; and
S10212, after enabling a first telescopic end 113 of the first coil 110 to pass through the fourth threading hole 2014 from the second side (not labeled in figure) of the fixing plate 200, movably connecting the first telescopic end 113 to the first fixed end 114 of the first coil 110 on the first side 202 of the fixing plate to form a first adjustable portion 115, thus making the first coil 110 form a first fixing portion 111 and a second fixing portion 112 for fixing a graft (not shown in figure) on the second side (not shown in figure) of the fixing plate 200.

In some further specific embodiments, as shown in FIG. 3, the first coil 110 forms one fixing portion 117 on the first side 202 of the fixing plate 200 by sequentially passing through the first threading hole 2011 and the second threading hole 2012 so as to achieve the connection to the fixing plate 200. The first telescopic end 113 and the first fixed end 114 are configured to respectively pass through the fourth threading hole 2014 and the third threading hole 2013. Further, at least one threading hole is further provided between the first threading hole 2011 and the second threading hole 2012, between the second threading hole 2012 and the third threading hole 2013, or between the third threading hole 2013 and the fourth threading hole 2014, thus increasing the friction force between the suture and the fixing plate, and unnecessary details are not given herein for the specific arrangement.

In still some specific embodiments, when the first coil forms at least one fixing portion on the first side of the fixing plate by passing through the plurality of threading holes so as to achieve the connection to the fixing plate, the number of the threading holes is two, respectively defined as a first threading hole and a second threading hole; the first coil forms one fixing portion on the first side of the fixing plate by sequentially passing through the first threading hole and the second threading hole so as to achieve the connection to the fixing plate.The first fixed end and the first telescopic end are configured to respectively pass through the first threading hole and the second threading hole and are then movably connected to form the first adjustable portion, and two ends of the first fixing portion respectively pass through the first threading hole and the second threading hole, and two ends of the second fixing portion respectively pass through the first threading hole and the second threading hole. Further, at least one threading hole is further provided between the first threading hole and the second threading hole, so that the first threading hole and the second threading hole, through which the two ends of the fixing portion penetrate, are provided at intervals, thus increasing the friction force between the suture and the fixing plate, and unnecessary details are not given herein for the specific arrangement.

In yet some specific embodiments of the present invention, when the first coil forms at least one fixing portion on the first side of the fixing plate by passing through the plurality of threading holes so as to achieve the connection to the fixing plate, the number of the threading holes is three, respectively defined as a first threading hole, a second threading hole, and a third threading hole. The first coil forms one fixing portion on the first side of the fixing plate by sequentially passing through the first threading hole and the second threading hole so as to achieve the connection to the fixing plate, i.e., one end of the first fixing portion passes through the first threading hole, one end of the second fixing portion passes through the second threading hole, and the other end of the first fixing portion passes through the second threading hole or the third threading hole, that is, the first telescopic end passes through the second threading hole or the third threading hole, the other end of the second fixing portion passes through the first threading hole or the third threading hole. And the threading hole, through which the other end of the second fixing portion passes, is different from the threading hole, through which the other end of the first fixing portion passes, that is, the first fixed end passes through the first threading hole or the third threading hole, and the threading hole through which the first fixed end passes is different from the threading hole through which the first telescopic end passes, and unnecessary details are not given herein for the specific arrangement.

In yet some specific embodiments of the present invention, when the first coil forms at least one fixing portion on the first side of the fixing plate by passing through the plurality of threading holes so as to achieve the connection to the fixing plate, the number of the threading holes is four, respectively defined as a first threading hole, a second threading hole, a third threading hole, and a fourth threading hole. The first coil forms two fixing portions on the first side of the fixing plate by sequentially passing through the first threading hole, the second threading hole, the third threading hole and the fourth threading hole so as to achieve the connection to the fixing plate. The first fixed end and the first telescopic end are configured to respectively pass through different threading holes, two ends of the first fixing portion respectively pass through different threading holes, the two ends of the second fixing portion respectively pass through different threading holes, and unnecessary details are not given herein for the specific arrangement.

In some embodiments of the present invention, the suture is further wound to form a second coil, the first coil forms a nested structure with the second coil so as to achieve the connection to the fixing plate; and the first coil and the second coil are woven by one continuous suture passing through the threading holes of the fixing plate, then the step S1 specifically comprises:
S111, enabling the continuous suture to pass through the threading holes and to form the first coil on the second side of the fixing plate, and movably connecting the first telescopic end of the first coil to the first fixed end of the first coil to form the first adjustable portion; and
S112, enabling the first fixed end to pass through the threading hole from the first side of the fixing plate and to pass through the first coil to form the nested structure, then winding the first fixed end to form the second coil; movably connecting the second telescopic end of the second coil to the second fixed end of the second coil to form the second adjustable portion or fixed connection, thus making the first coil form the first fixing portion and the second fixing portion for fixing the graft on the second side of the fixing plate.

Specifically, the step S111 comprises: enabling the suture to pass through the threading holes and forming the first coil on the second side of the fixing plate, and then movably connecting the first telescopic end of the first coil to the first fixed end of the first coil to form the first adjustable portion; or firstly winding the suture to form the first coil and then movably connecting the first telescopic end of the first coil to the first fixed end of the first coil to form a first adjustable portion, and finally providing the first coil on the second side (not labeled in figure) of the fixing plate by passing through the second threading hole.

In some specific embodiments of the present invention, the suture is further wound to form a second coil, when the first coil forms a nested structure with the second coil so as to achieve the connection to the fixing plate, and the first coil and the second coil are woven by one continuous suture passing through the threading holes of the fixing plate, the first telescopic end and the first fixed end pass through the same or common threading hole, the second telescopic end and the second fixed end pass through the same or common threading hole, and at the moment, the threading hole through which the second telescopic end and the second fixed end pass is different from the threading hole through which the first telescopic end and the first fixed end pass.

In some further specific embodiments of the present invention, the suture is further wound to form a second coil, the first coil forms a nested structure with the second coil so as to achieve the connection to the fixing plate, and the first coil and the second coil are woven by one continuous suture passing through the threading holes of the fixing plate, the first telescopic end and the first fixed end are configured to pass through the same or common threading hole, the second telescopic end and the second fixed end are configured to pass through different threading holes. And at the moment, the threading hole through which the second telescopic end passes and the threading hole through which the second fixed end passes may be the same as or different from the threading hole through which the first telescopic end and the first fixed end pass.

In still some specific embodiments of the present invention, the suture is further wound to form a second coil, the first coil forms a nested structure with the second coil so as to achieve the connection to the fixing plate, and the first coil and the second coil are woven by one continuous suture passing through the threading holes of the fixing plate, wherein the first telescopic end and the first fixed end pass through different threading holes, the second telescopic end and the second fixed end pass through the same threading holes. And at the moment, the threading hole through which the first telescopic end passes and the threading hole through which the first fixed end passes may be the same as or different from the threading hole through which the second telescopic end and the second fixed end pass.

In yet some specific embodiments of the present invention, the suture is further wound to form a second coil, the first coil forms a nested structure with the second coil so as to achieve the connection to the fixing plate, and the first coil and the second coil are woven by one continuous suture passing through the threading holes of the fixing plate, wherein the first telescopic end and the first fixed end pass through different threading holes, the second telescopic end and the second fixed end pass through different threading holes. And at the moment, the threading hole through which the second telescopic end passes and the threading hole through which the second fixed end passes may be the same as or different from the threading hole through which the first telescopic end passes, and the threading hole through which the second telescopic end passes and the threading hole through which the second fixed end passes may be the same as or different from the threading hole through which the first fixed end passes.

FIG. 14 is a structure diagram formed in a manufacturing process of a medical loop of an embodiment of the present invention.

In some specific embodiments of the present invention, referring to FIG. 6 and FIG. 14, a manufacturing method of a medical loop is provided, comprising:
S11100, providing a fixing plate 200 and one continuous suture (not labeled in figure), wherein the fixing plate 200 is provided with two threading holes, and the threading holes 201 are respectively defined as a first threading hole 2011 and a second threading hole 2012;
S11101, enabling the continuous suture (not labeled in figure) to partially pass through the second threading hole 2012 and forming a first coil 110 on the second side (not shown in figure) of the fixing plate 200, and movably connecting a first telescopic end 113 of the first coil 110 and a first fixed end 114 of the first coil 110 to form a first adjustable portion 115; and
S11202, enabling the first fixed end 114 from a first side 202 of the fixing plate 200 through the second threading hole 2012 to the second side (not labeled in figure) of the fixing plate 200 to form a second coil 120, which forms a nested structure 130 with the first coil; movably connecting the second telescopic end 121 of the second coil 120 to the second fixed end 122 of the second coil 120 to form a second adjustable portion 123, thus making the first coil 110 form a first fixing portion 111 and a second fixing portion 112 for fixing a graft (not labeled in figure) on the second side (not labeled in figure) of the fixing plate 200.

In some other specific embodiments of the present invention, referring to FIG. 7 and FIG. 14, a manufacturing method of a medical loop is provided, comprising:
S11110, providing a fixing plate 200 and one continuous suture (not labeled in figure), wherein the fixing plate 200 is provided with three threading holes 201, and the threading holes 201 are respectively defined as a first threading hole 2011, a second threading hole 2012 and a third threading hole 2013 arranged in sequence;
S11111, enabling the continuous suture (not labeled in figure) to pass through the third threading hole 2013 and forming a first coil 110 on a second side (not labeled in figure) of the fixing plate 200, and movably connecting a first telescopic end 113 of the first coil 110 to a first fixed end 114 of the first coil 110 to form a first adjustable portion 115; and
S11212, enabling the first fixed end 114 from a first side 202 of the fixing plate 200 through the second threading hole 2012 to the second side (not labeled in figure) of the fixing plate 200 through the first coil 110 to form a second coil 120, which has a nested structure 130 with the first coil 110; enabling a second telescopic end 121 of the second coil 120 to pass through the first threading hole 2011 to the first side 202 of the fixing plate 200 and movably connecting the second telescopic end 121 of the second coil 120 to a second fixed end 122 of the second coil 120 to form a second adjustable portion 123, thus making the first coil 110 form a first fixing portion 111 and a second fixing portion 112 for fixing a graft (not labeled in figure) on the second side (not labeled in figure) of the fixing plate 200.

In some further embodiments of the present invention, referring to FIG. 8 and FIG. 14, a manufacturing method of a medical loop is provided, comprising:
S11120, providing a fixing plate 200 and one continuous suture (not labeled in figure), wherein the fixing plate 200 is provided with four threading holes 201, and the threading holes 201 are respectively defined as a first threading hole 2011, a second threading hole 2012, a third threading hole 2013 and a fourth threading hole 2014 arranged in sequence;
S11121, enabling one end of the continuous suture (not labeled in figure) to pass through the fourth threading hole 2014 to form a first coil 110 on a second side (not labeled in figure) of the fixing plate 200, threading a first fixed end 114 of the first coil 110 to a first side 202 of the fixing plate 200 through the third threading hole 2013, and then movably connecting a first telescopic end 113 of the first coil 110 to a first fixed end 114 to form a first adjustable portion 115; and
S11222, enabling the first fixed end 114 from the first side 202 of the fixing plate 200 through the second threading hole 2012 to the second side (not labeled in figure) of the fixing plate 200 to form a second coil 120,which forms a nested structure 130 with the first coil 110; enabling a second telescopic end 121 of the second coil 120 to pass through the first threading hole 2011 to the first side 202 of the fixing plate 200, and movably connecting the second telescopic end 121 of the second coil 120 to a second fixed end 122 of the second coil 120 to form a second adjustable portion 123, thus making the first coil 110 form a first fixing portion 111 and a second fixing portion 112 for fixing a graft (not labeled in figure) on the second side (not shown in figure) of the fixing plate 200.

In some embodiments of the present invention, the two sutures of the at least one suture are respectively wound to form the first coil and the second coil, the first coil is woven by one of the sutures according to the step S111, and after the first adjustable portion is formed, the following steps are executed sequentially:
S122, threading one end of the other of the sutures through the first coil in the step S111 to form the nested structure, and then winding the end of the other of the sutures to form the second coil; and
S123, movably connecting the second telescopic end of the second coil to the second fixed end of the second coil to form a second adjustable portion or fixed connection, thus making the first coil form the first fixing portion and the second fixing portion for fixing the graft on the second side of the fixing plate.

In some specific embodiments of the present invention, when two sutures of the at least one suture are respectively wound to form the first coil and the second oil, the first telescopic end and the first fixed end are configured to pass through different threading holes, the second telescopic end and the second fixed end are configured to pass through different threading holes, wherein the threading hole through which the second telescopic end passes and the threading hole through which the second fixed end passes may be the same as or different from the threading hole through which the first telescopic end passes, and the threading hole through which the second telescopic end passes and the threading hole through which the second fixed end passes may be the same as or different from the threading hole through which the first fixed end passes.

In some other embodiments of the present invention, after the step S123, the manufacturing method further comprises: fixedly connecting the second fixed end to the first fixed end; at the moment, in some specific embodiments, the threading hole through which the second fixed end passes is different from the threading hole through which the first fixed end passes, while the threading hole through which the first telescopic end passes may be the same as or different from the threading hole through which the first fixed end passes, and the threading hole through which the second telescopic end passes may be the same as or different from the threading hole through which the second fixed end passes. In some other specific embodiments of the present invention, the threading hole through which the second fixed end passes is the same as the threading hole through which the first fixed end passes, the threading hole through which the first telescopic end passes is different from the threading hole through which the first fixed end passes, and the threading hole through which the second telescopic end passes may be the same as or different from the threading hole through which the second fixed end passes; in some further specific embodiments of the present invention, the threading hole through which the second fixed end passes is the same as the threading hole through which the first fixed end passes, the threading hole through which the second telescopic end passes is different from the threading hole through which the second fixed end passes, and the threading hole through which the first telescopic end passes may be the same as or different from the threading hole through which the first fixed end passes.

In some specific embodiments of the present invention, referring to FIG. 9 and FIG. 14, a manufacturing method of a medical loop is provided, comprising:
S1200: providing a fixing plate 200 and two sutures (not labeled in figure), wherein the fixing plate 200 is provided with four threading holes 201, and the threading holes 201 are respectively defined as a first threading hole 2011, a second threading hole 2012, a third threading hole 2013 and a fourth threading hole 2014 arranged in sequence;
S1211, after threading one end of one of the sutures (not labeled in figure) through the third threading hole 2013 and forming a first coil 110 on a second side (not labeled in figure) of the fixing plate 200, threading a first telescopic end 113 of the fist coil 110 to a first side 202 of the fixing plate 200 through the fourth threading hole 2014, and then movably connecting the first telescopic end 113 to a first fixed end 114 of the first coil 110 to form a first adjustable portion 115;
S1222, after threading one end of the other of the sutures (not labeled in figure) through the first coil 110 in the step S1211 to form a nested structure 130, winding the end of the other of the sutures into a second coil 120; and
S1233, enabling a second telescopic end (not labeled in figure) of the second coil 120 and a second fixed end (not labeled in figure) of the second coil 120 to the first side 202 of the fixing plate 200 to respectively pass through the third threading hole 2013 and the fourth threading hole 2014, fixedly connecting the second telescopic end (not labeled in figure) of the second coil 120 to the second fixed end (not labeled in figure) of the second coil 120, thus making the first coil 110 form a first fixing portion 111 and a second fixing portion 112 for fixing a graft on the second side (not labeled in figure) of the fixing plate 200.

While the embodiments of the present invention have been described in detail, it will be apparent to those skilled in the art that various modifications and variations can be made to the embodiments. However, it is to be understood that such modifications and variations are within the scope and spirit of the present invention as described in the appended claims. Furthermore, the present invention described herein is susceptible to other embodiments and may be embodied or carried out in various ways.

## Claims

1. A medical loop, comprising a fixing plate and at least one suture, the fixing plate is provided with at least two threading holes, and the suture is wound to form a first coil wherein two free ends of the first coil are configured to pass through the threading holes, and the first coil is connected to the fixing plate to form a first fixing portion and a second fixing portion adapted to fix a graft; the two free ends of the first coil respectively defined as a first telescopic end and a first fixed end, the first telescopic end and the first fixed end configured to be movably connected therewith to form a first adjustable portion.

2. The medical loop according to claim 1, wherein the suture is further wound to form a second coil, two free ends of the second coil are respectively defined as a second telescopic end and a second fixed end, the second telescopic end and the second fixed end configured to pass through the threading holes, and the first coil forms a nested structure with the second coil so as to achieve the connection to the fixing plate.

3. The medical loop according to claim 2, wherein the at least one suture comprises a continuous suture, the first coil and the second coil are woven by the continuous suture passing through the threading holes of the fixing plate.

4. The medical loop according to claim 2, wherein the first coil and the second coil are respectively woven by two sutures of the at least one continuous suture to pass through the threading holes of the fixing plate.

5. The medical loop according to claim 4, wherein the first fixed end is fixedly connected to the second fixed end.

6. The medical loop according to claim 3 or 5, wherein the first telescopic end and the first fixed end are configured to pass through the common threading hole.

7. The medical loop according to claim 1 or 2, wherein the first telescopic end and the first fixed end are configured to respectively pass through the different threading holes, and the first telescopic end and the first fixed end are movably connected therewith at the first side of the fixing plate to form the first adjustable portion.

8. The medical loop according to claim 3 or 5, wherein the second telescopic end and the second fixed end are configured to pass through the common threading hole, and the second telescopic end and the second fixed end are movably connected therewith to form a second adjustable portion.

9. The medical loop according to claim 3 or 5, wherein the second telescopic end and the second fixed end are configured to pass through the common threading hole, and the second telescopic end is fixedly connected to the second fixed end.

10. The medical loop according to claim 2, wherein the second telescopic end and the second fixed end are configured to respectively pass through the different threading holes, and the second telescopic end and the second fixed end are movably connected therewith at the first side of the fixing plate to form the second adjustable portion.

11. The medical loop according to claim 2, wherein the second telescopic end and the second fixed end are configured to respectively pass through the different threading holes, and the second telescopic end is fixedly connected to the second fixed end.

12. The medical loop according to claims 2, wherein the second telescopic end and the second fixed end are movably connected therewith by way of any one of a threading process, a nesting method, a fastening method, a clamping method, and a binding method to form the second adjustable portion.

13. The medical loop according to claim 1, wherein the first coil is configured to form at least one fixing portion on the first side of the fixing plate by passing through the threading holes, thus achieving the connection to the fixing plate.

14. The medical loop according to claim 1, wherein the first telescopic end and the first fixed end are movably connected therewith by way of any one of a threading process, a nesting method, a fastening method, a clamping method and a binding method to form the first adjustable portion.

15. A manufacturing method of the medical loop according to any one of claims 1-14, comprising:
S0, providing a fixing plate and at least one suture, wherein the fixing plate is provided with at least two threading holes; and
S1, winding the suture to form a first coil, enabling two free ends of the first coil to pass through the threading holes, and connecting the first coil to the fixing plate to form the first fixing portion and the second fixing portion adapted to fix the graft, and enabling a first telescopic end of the first coil and a first fixed end of the first coil to be movably connected therewith to form a first adjustable portion.

16. The manufacturing method of the medical loop according to claim 15, wherein the first coil is configured to form at least one fixing portion on the first side of the fixing plate by passing through the threading holes, thus achieving the connection to the fixing plate, then the step S1 specifically comprises:
S101, enabling one free end of the suture to pass through the threading holes to form at least one fixing portion on the first side of the fixing plate so as to achieve the connection to the fixing plate, and forming the first coil on the second side of the fixing plate; and
S102, enabling the first telescopic end of the first coil and the first fixed end of the first coil to be movably connected therewith to form the first adjustable portion,which is located on the first side of the fixing plate, thus making the first coil form the first fixing portion and the second fixing portion adapted to fix the graft on the second side of the fixing plate.

17. The manufacturing method of the medical loop according to claim 15, wherein the at least one suture is further wound to form the second coil, and the first coil is configured to form a nested structure with the second coil to achieve the connection to the fixing plate, and the first coil and the second coil are woven by a continuous suture through the threading holes of the fixing plate, then the step S1 specifically comprises:
S 111, enabling the continuous suture to pass through the threading holes and to form the first coil on the second side of the fixing plate, and movably connecting the first telescopic end of the first coil to the first fixed end of the first coil to form the first adjustable portion; and
S112, enabling the first fixed end to pass through the threading hole from the first side of the fixing plate and further to pass through the first coil to form the second coil, which is configured to form the nested structure with the first coil; movably connecting the second telescopic end of the second coil to the second fixed end of the second coil to form the second adjustable portion or fixed connection, thus making the first coil form the first fixing portion and the second fixing portion adapted to fix the graft on the second side of the fixing plate.

18. The manufacturing method of the medical loop according to claim 17, wherein enabling two sutures of the at least one suture to be respectively wound to form the first coil and the second coil, wherein the first coil is woven by one of the sutures according to the step S111, and when the first adjustable portion is formed, the following steps are executed sequentially:
S122, enabling one free end of the other of the sutures to pass through the first coil of the step S111 to form the second coil, which is configured to form the nested structure with the first coil; and
S123, movably connecting the second telescopic end of the second coil to the second fixed end of the second coil to form the second adjustable portion or fixed connection, thus making the first coil form the first fixing portion and the second fixing portion adapt to fix the graft on the second side of the fixing plate.
